# EUROPEAN PATENT APPLICATION

(11) **EP 2 368 627 A1**
(43) Date of publication of application: **28.09.2011**
(21) Application number: 09834970.7
(22) Date of filing: 24.12.2009
(51) Int. Cl.: B01J 13/04, A61K 8/14, A61K 8/55, A61K 8/63, A61K 9/127

(54) **METHOD FOR PRODUCING LIPOSOME AND METHOD FOR DISSOLVING CHOLESTEROL**

(30) Priority: 24.12.2008 JP 2008328724
(71) Applicant: Biomedcore Inc., Kanagawa 236-0004 (JP)
(72) Inventor: SHIMIZU, Yoshitaka, Yokohama-shi Kanagawa 236-0005 (JP); MIBE, Yasuhiko, Yokohama-shi Kanagawa 241-0812 (JP)
(74) Representative: Grünecker, Kinkeldey, Stockmair & Schwanhäusser Anwaltssozietät
(86) International application number: PCT/JP2009/071484
(87) International publication number: WO 2010/074172

(57) **Abstract**

Methods are provided for producing liposomes, including a first mixing step of mixing one or more lipids and an aqueous solution containing a water-miscible organic solvent; a heating step of heating the mixture of the lipids and the aqueous solution to a temperature at which the lipids are dissolved in the aqueous solution; and, after the heating step, a cooling step of cooling the resulting mixture to a temperature at which liposomes are produced. By this method, liposomes with a uniform particle diameter and have a high encapsulation ratio can be easily obtained.

## Description

### CROSS REFERENCE TO RELATED APPLICATIONS

This application claims the benefit of priority to Japanese Patent Application No. 2008-328724, filed on December 24, 2008, which is incorporated herein by reference.

### TECHNICAL FIELD

The present invention relates to methods for producing liposomes and methods for dissolving cholesterol.

### BACKGROUND ART

A liposome is a roughly spherical hollow particle enclosed by at least one lipid bilayer composed of lipid molecules. The lipid molecule possesses hydrophilic groups having hydrophilicity and, on the opposite side, lipophilic groups having lipophilicity. For this reason, in contact with water, the molecules form a bilayer, which becomes spherical so that the surface area is minimal, the hydrophilic groups facing outside the bilayer as well as inward toward the inner compartment formed by the bilayer, while the lipohilic groups pointing toward the interior of the bilayer.

Since the bilayer thus forming a liposome is similar to the cell membrane constituting the living body, it is easily accepted in the biological environment. In recent years, by taking advantage of this property, liposomes have been a focus of attention as pharmaceutical vesicles in the drug delivery system (DDS), in which a drug encapsulated in the region surrounded by the bilayer is transported to the site in the living body requiring the medicine. Another use of liposomes, for example, cosmetic microcapsules encapsulating cosmetics has drawn attention.

Methods for producing liposomes include various techniques such as the reverse-phase evaporating method, sonication method, extrusion method, French press method, homogenization method, ethanol injection method, dehydration-rehydration method, of which one typical technique is the Bangham method (thin-film method). In this method, a suspension containing liposomes is obtained as follows: at least one phospholipid is dissolved in an organic solvent, such as chloroform, in a vessel, such as a flask; then by evaporating off chloroform, lipid membrane is temporarily formed at the bottom of the vessel, to which an aqueous solution, such as buffer, is added and the vessel is mixed (A. D. Bangham et al. , J. Mol. Biol., 13, 238-252 (1965); A. D. Bangham and R. W. Horne, J. Mol. Biol., 8, 660-668 (1964).

Alternatively, typical industrial manufacturing methods for liposomes include the technique in which lipid components, such as phospholipid dissolved in a water-miscible organic solvent, is added to an aqueous solution by infusion with stirring. The water-miscible organic solvents which can be suitably used here include alcohols, such as methanol, ethanol, isopropyl alcohol, and butanol. It should be noted, however, that the lipid solution should be added to and mixed with an aqueous solution while being warmed so that the dissolved state of the lipid is maintained; this requires precise control of temperature, adding speed or stirring speed (National Publication of International Patent Application No. 2006-517594).

Furthermore, another method of producing a preliposome using t-butanol has also been reported (National Publication of International Patent Application No.1994-509547) This method has an advantage that lipid can be sterilized by filtration and be freeze-dried, since lipid can be maintained as a solution around room temperature by using, as a dissolution solvent of lipid, t-butanol having a water content as low as about 20%.

### DISCLOSURE OF THE INVENTION

### PROBLEMS TO BE SOLVED BY THE INVENTION

However, the above-mentioned conventional methods and the liposomes produced thereby have the following problems: liposomes are not uniform in particle diameter or liposomes uniform in particle diameter cannot be easily produced. Both liposomes with a small particle diameter (small unilameller vesicles: SUVs) and those with a large particle diameter (large unilameller vesicles: LUVs) have their use value; however, if the particles are mix of them, their use value decreases. For that reason, liposomes having a wide distribution of their particle sizes are passed through a filter having pores to narrow the distribution. However, such a treatment causes the problem of a low yield of liposomes having a certain particle size required. Meanwhile, in order to increase the membrane strength of the lipid bilayer, the method of incorporating cholesterol in the lipid bilayer is known. However, since cholesterol is generally difficult to be dissolved in water-miscible organic solvents, a method for dissolving cholesterol has been in demand.

The present invention has been achieved in view of the above-described problems. An object of the present invention is to provide a method whereby liposomes uniform in particle diameter can be easily obtained. Another object of the present invention is to provide a method for increasing the solubility of cholesterol.

### MEANS FOR SOLVING THE PROBLEM

The inventors have assiduously studied in order to achieve the above objects, and, as a result, found that liposomes uniform in particle diameter can be easily produced by mixing one or more lipids and an aqueous solution containing a water-miscible organic solvent, heating the resulting mixture, and cooling the mixture to a given temperature. They have also found that, by mixing cholesterol with a phospholipid and an aqueous solution containing a water-miscible organic solvent and heating the resulting mixture, cholesterol can be easily dissolved in the aqueous solution. Thus, the inventors have accomplished the present invention.

That is, the method for producing a liposome according to the present invention includes the steps of heating a mixture containing one or more lipids, water and an organic solvent; and cooling the mixture after the heating step.

The mixture is, for example, a mixture obtained by mixing the lipid and the aqueous solution containing a water-miscible organic solvent.

Preferably, the aqueous solution contains 5 to 30% by volume of the water-miscible organic solvent, relative to the total volume of the solution.

The exemplary water-miscible organic solvent that can be used includes one or more organic solvents or the like selected from t-butanol, 1-propanol, 2-propanol, and 2-butoxyethanol.

Further, the method for dissolving cholesterol according to the present invention includes the step of heating a mixture obtained by mixing cholesterol with a phospholipid, water and an organic solvent.

Further, the method for dissolving cholesterol according to the present invention includes the step of heating a mixture obtained by mixing cholesterol with a phospholipid and an aqueous solution comprising a water-miscible organic solvent.

Preferably, the aqueous solution contains 5 to 30% by volume of the water-miscible organic solvent, relative to the total volume of the solution.

The exemplary water-miscible organic solvent that can be used includes one or more organic solvents or the like selected from t-butanol, 1-propanol, 2-propanol, and 2-butoxyethanol.

Further, the method for selecting an organic solvent, according to the present invention, which can be used for producing a liposome includes the step of producing a liposome by using the above-mentioned methods with a candidate organic solvent; and the step of examining whether or not the liposome are uniform

### Effect of the Invention

According to the present invention, there can be provided methods whereby liposomes uniform in particle diameter can be easily obtained. Further, according to the present invention, there can be provided methods for improving the solubility of cholesterol.

### BRIEF DESCRIPTION OF THE DRAWINGS

FIG. 1 is a particle size distribution chart of the liposome suspension prepared under the conditions of a t-butanol (t-BuOH) concentration of 16 vol% in Experimental Example according to the present invention.
FIG. 2 shows the result of the particle size distribution obtained when liposome suspensions were prepared from aqueous solutions containing each water-miscible organic solvent using dipalmitoylphosphatidylcholine (DPPC), cholesterol, and stearylamine in an Example according to the present invention.
FIG. 3 shows the result of the particle size distribution obtained when liposome suspensions were prepared from aqueous solutions containing each water-miscible organic solvent using DPPC, cholesterol, and dipalmitoylphosphatidylglycerol (DPPG) in an Example according to the present invention.

### EMBODIMENTS FOR CARRYING OUT THE INVENTION

The following is a description of the preferred embodiments of the method for producing liposomes and method for dissolving cholesterol according to the present invention. The embodiments of the method for dissolving cholesterol will be described within the embodiments of the method for producing liposomes.

### (1) Liposome

Liposomes include empty liposomes which contain no physiologically active substance such as a pharmaceutical agent in their vesicle, and liposomes encapsulating physiologically active substances in the vesicle. Liposomes are also classified as vesicles with relatively small particles (small unilameller vesicles: SUVs) formed by a single lipid bilayer and vesicles with relatively large particles formed by a single lipid bilayer (large unilameller vesicles: LUVs) as well as vesicles formed by multiple membrane layers (multi-lameller vesicles: MLVs). Liposomes of any particle size may be used as the liposome of the present invention, but preferred mean particle diameter is 50 to 2000 nm, particularly preferred is 100 to 700 nm. The term "particle diameter" herein refers to the diameter of a particle measured by dynamic light scattering. The preferred polydispersity index (PDI) is 0.3 or less.

As physiologically active substances that can be encapsulated in the liposome, various pharmaceutical agents, cosmetics, and the like can be adopted. Such substances are exemplified by one or a combination of anticancer agents such as cisplatin, 5-fluorouracil, etc. as well as antioxidants, antibacterial agents, anti-inflammatory agents, blood circulation-promoting agents, anti-aging agents, hormone formulation, vitamin formulation, hemoglobin, DNA, RNA, peptides, protein, vaccines, hair-growing agents, moisturizers, coloring agents, whitening agents, pigments, saline, water, etc. However, the physiologically active substances are not limited to the above examples. Moreover, the surface of the liposome may be modified with a functional group or the like. Such modification with a functional group can be realized either by binding a functional group to the phospholipid or the like before or after liposome formation.

### (2) Method for producing liposomes

Any known method may be applied to the method for producing liposomes according the embodiments of the present invention, as long as the method includes the steps of heating a mixture obtained by mixing one or more lipids and an aqueous solution containing a water-miscible organic solvent, and cooling the mixture after the heating step. More specifically, in order to adjust the particle diameter of liposomes, the sonication method, extrusion method, French press method, homogenization method, etc. may be combined with the methods according to the present invention

In the method for producing liposomes according to the present invention, one or more lipids such as soybean lecithin, hydrogenated soybean lecithin, egg yolk lecithin, phosphatidylcholines, phosphatidylserines phosphatidylethanolamines, phosphatidyl inositols, sphingomyelins, phosphatidic acids, long-chain alkyl phosphates, gangliosides, glycolipids, phosphatidyl glycerols, and sterols, can be used. Phosphatidylcholines can be exemplified by dimyristoylphosphatidylcholine, dipalmitoylphosphatidylcholine, distearoylphosphatidylcholine, etc. Phosphatidylserines can be exemplified by dipalmitoyl phosphatidylserine, dipalmitoyl phosphatidylserine (sodium salt), phosphatidylserine (sodium salt) derived from bovine brain, etc. Phosphatidylethanolamines can be exemplified by dimyristoylphosphatidylethanolamine, dipalmitoylphosphatidylethanolamine, distearoylphosphatidylethanolamine, etc. Phosphatidyl inositols can be exemplified by phosphatidylinositol (sodium salt) derived from wheat, etc. Sphingomyelins can be exemplified by sphingomyelin derived from bovine brain etc. Phosphatidic acids and long-chain alkyl phosphates are exemplified by dimyristoyl phosphatidic acid, dipalmitoyl phosphatidic acid, distearoyl phosphatidic acid, dicetyl phosphate, etc. Gangliosides are exemplified by ganglioside GM1 and ganglioside GD1a, ganglioside GT1b, etc. Glycolipids are exemplified by galactosyl ceramide, glucosyl ceramide, lactosyl ceramide, phosphatide, globoside, etc. Phosphatidyl glycerols can be exemplified by dimyristoylphosphatidylglycerol, dipalmitoylphosphatidylglycerol, distearoylphosphatidylglycerol, etc. Sterols can be exemplified by cholesterol, dihydrocholesterol, lanosterol, dihydrolanosterol, sitosterol, campesterol, stigmasterol, brassicasterol, ergosterol, phytosterol (the mixture thereof), and hydrogenated phytosterol, as well as positively-charged sterols such as 3β-[N-(dimethylaminoethane)carbamoyl]cholesterol and N-(trimethylammonio ethyl)carbamoyl cholesterol. The preferred lipid that constitutes the liposome is a combination of a phospholipid containing phosphorus and cholesterol. In particular, a combination of phosphatidylcholine that is a type of phospholipid and cholesterol is more preferred. Where a liposome is produced using a phospholipid and cholesterol, the molar ratio of the phospholipid to cholesterol is preferably in the range of 1:0 to 1:1.5 and more preferably in the range of 1:0.5 to 1:1.25.

The term "water-miscible organic solvent" as used herein refers to an organic solvent that can be misced with water, exemplified by alcohols, ethers, esters, ketones, and acetals. A preferred water-miscible organic solvent to be used is one or more organic solvents selected from t-butanol, 1-propanol, 2-propanol, and 2-butoxyethanol. Mixing can be performed by methods using manual shaking, stirring with a stirring bar and an stirring blade, use of an ultrasonic vibrator, or the like.

As for the concentration of the water-miscible organic solvent in the above-described aqueous solution, the optimal concentration must be selected, depending on the lipid composition and lipid concentration. This is because when increasing the concentration of the water-miscible organic solvent, the solubility of the lipid is increased, but as a result liposomes are not formed. Moreover, at the same time, since the water-miscible organic solvent easily remains with the liposomes, they can exert an unfavorable effect on the living body into which they have been introduced. Therefore, preferably, the concentration of the water-miscible organic solvent in the aqueous solution is the lowest possible to dissolve one or more lipids in the solution by mixing the solution with the lipids and heating the mixture. Specifically, the concentration of the water-miscible organic solvent is preferably 5 to 30% by volume, relative to the total volume of the aqueous solution. Where the water-miscible organic solvent is t-butanol, the concentration is particularly preferably 12 to 18% by volume, relative to the total volume of the aqueous solution. Where the water-miscible organic solvent is 1-propanol, the concentration is particularly preferably 5 to 19% by volume, relative to the total volume of the aqueous solution. Where the water-miscible organic solvent is 2-propanol, the concentration is particularly preferably 13 to 26% by volume, relative to the total volume of the aqueous solution. Where the water-miscible organic solvent is 2-butoxyethanol, the concentration is particularly preferably 6 to 9% by volume, relative to the total volume of the aqueous solution.

Additionally, an aqueous solution prepared in advance so as to contain the above-mentioned water-miscible organic solvent at the above-mentioned concentration may be added to the lipids. Alternatively, an aqueous solution may be added such that it contains the above-mentioned water-miscible organic solvent at the above mentioned concentration after once the lipid moiety has been temporarily dissolved by adding the water-miscible organic solvent.

The heating method is not particularly limited, and the warm water bath method for immersing the vessel in a water bath with warm water, method for heating a vessel with the mixture over direct fire, the method for placing the vessel in an electric heater, or the like can be adopted. The heating temperature is not particularly limited as long as it is at or above the temperature at which the lipid is dissolved in the aqueous solution containing a water-miscible organic solvent and at which the aqueous solution is not white opaque. The heating temperature varies depending on the type of the lipid, the concentration of the lipid, the type of the water-miscible organic solvent, etc., but, in general, it is preferably in the range of 62 to 80°C, particularly preferably in the range of 65 to 72°C. It should be noted that where t-butanol serves as the water-miscible organic solvent and phosphatidylcholine as well as cholesterol serve as the lipids, the heating temperature is preferably in the range of 62 to 72°C.

The cooling method is not particularly limited, but the method for immersing the vessel in a water bath with cold water, method for placing the vessel with the mixture in the refrigerator, or the like can be adopted. The cooling temperature is not limited as long as it is lower than the heating temperature and liposomes are formed. For example, where phosphatidylcholine and cholesterol are used as the lipids, the cooling temperature is preferably 62°C or lower. In particular, preferably, the mixture is cooled to 62°C or lower after being heated at 72°C or higher. The cooling rate is preferably 0.5°C/min or more, and is more preferably 1°C/min or more.

When mixing the lipid with the aqueous solution containing a water-miscible organic solvent, sugar such as a disaccharide or a polysaccharide may be added as an osmotic pressure-controlling agent. A preferred sugar is sucrose, which is a disaccharide. Sucrose can also be mixed with the aqueous solution containing a water-miscible organic solvent in the state of an aqueous solution. In such a case, a preferred concentration of the sucrose aqueous solution is 5 to 70 wt/vol%, and more preferred is 8 to 50 wt/vol%.

In the method for producing liposomes according to the present invention, in order to render the particles in the liposome suspension more uniform in particle size, a known sizing means may be concurrently used. For example, liposomes with a desirable pore size may be obtained by passing the liposome suspension through a membrane with the specific pore size using gas pressure. Such a process of passing liposomes through a menbrane with the specific pore size may be performed once or multiple times.

### (3) Method for dissolving cholesterol (Method for increasing the amount of dissolved cholesterol relative to an aqueous solution containing a water-miscible organic solvent)

Cholesterol, a kind of lipid, is hard to be dissolved in water under normal conditions, thus having a low rate of dissolution in the above-mentioned water-miscible organic solvent. However, in the co-presence of a phospholipid in the aqueous solution containing an above-mentioned water-miscible organic solvent, cholesterol becomes easy to be dissolved in water by heating. Specifically, by heating a mixture obtained by mixing a phospholipid and cholesterol in the above-mentioned aqueous solution, cholesterol can be easily dissolved in the aqueous solution.

The heating temperature is not limited as long as it is at or above the temperature at which cholesterol can be dissolved in an aqueous solution containing a phospholipid, a water-miscible organic solvent, etc. and at which the aqueous solution does not become white opaque. For example, where t-butanol and L-α-dipalmitoylphosphatidylcholine are used as the water-miscible organic solvent and the phospholipid, respectively, cholesterol can be sufficiently dissolved in the aqueous solution at a temperature of 62 to 72°C. Cholesterol is easier to be dissolved in the range of 0.8 to 1.5 mol, relative to 1 mol of a phospholipid. In the range of 62 to 72°C, cholesterol can be dissolved in an aqueous solution containing t-butanol, which is totally transparent. If cooling the solution below that range so that the temperature of the solution becomes below 62°C, the solution turns white opaque, resulting in formation of a suspension of a liposome composed of the phospholipid and cholesterol. On the contrary, if the temperature is above 72°C, the mixture also turns into a white opaque suspension.

### (4) Method for selecting usable organic solvents

By using the production methods described so far, liposomes with a uniform particle diameter can be obtained. Nevertheless, there are some organic solvents that cannot be applied. Thus, by producing liposomes by the above described liposome production methods using candidate organic solvents, and then examining if the liposome particles produced are uniform in size, it can be determined if the solvents are usable in the production methods. This procedure enables selection of practically applicable organic solvents.

### EXAMPLE

The Examples of the present invention will be described hereinbelow; however, the Examples should not be construed as limiting the present invention.

1. Materials for liposomes
   a) Phospholipid
      L-α-dipalmitoylphosphatidylcholine (DPPC) manufactured by Nippon Oil & Fats Co., Ltd. was used.
   b) Cholesterol
      Cholesterol (Chol.) manufactured by Sigma Chemical Co. was used.
   c) Stabilizing agent
      Sucrose manufactured by Wako Pure Chemical Industries, Ltd. was used.
   d) Water-miscible organic solvents
      t-Butanol (t-BuOH; special grade) manufactured by Wako Pure Chemical Industries, Ltd., 1-propanol (special grade) manufactured by Wako Pure Chemical Industries, Ltd., 2-propanol (special grade) manufactured by Wako Pure Chemical Industries, Ltd., and 2-butoxyethanol (special grade) manufactured by Wako Pure Chemical Industries, Ltd. were used.
   e) Lipids other than a) and b)
      Dipalmitoylphosphatidylglycerol (DPPG), dipalmitoyl phosphatidylethanolamine (DPPE), and hydrogenated soy phosphatidylcholine (HSPC) were purchased from Nippon Oil & Fats Co., Ltd. Stearylamine (SA) and dicetyl phosphate (DCP) were purchased from a Wako Pure Chemical Industries, Ltd. and Sigma Chemical Corporation, respectively.

### 2. Method for measuring particle size distribution of liposomes

Measurement of particle size distribution of the liposomes was performed using a particle size distribution measurement device (Zetasizer Nano ZS, manufactured by Malvern Instruments Ltd.) measured by dynamic light scattering. The liposomes prepared in the Example Experiment described later were subjected to size measurement after being diluted with phosphate buffered saline (PBS). The dilution was approximately 5000- to 10000-fold. The values measured with the Zetasizer Nano ZS were presented as the mean particle diameter, Z-Average (d. nm). Using the polydispersity index (PDI) values presented concurrently as the index, the uniformity of the liposome particle size distribution was evaluated.

Further, the results of the measurement displayed as "Result quality" on the above-mentioned particle size distribution measurement device were taken as the criteria for judging whether liposomes uniform in particle size had been formed. That is, when the liposomes met the quality standard defined by Malvern in particle diameter measurement, "Good" was displayed for "Result quality." When a "Good" was not displayed as a measurement result, it was judged that the samples were not uniform in particle size, unsuitable for dynamic light scattering.

### 3. Experiment Example

### 3.1: Examination of temperature ranges for dissolving phospholipid and cholesterol

380 mg of DPPC and 200 mg of cholesterol were weighed in a glass vial, to which was added a mixture of 20 mL of 10 wt/vol% sucrose solution and 4.25 mL of t-BuOH. This vial was stirred for 10 min in a water bath maintained at 80°C. The solution was in the white opaque state at 80°C. Next, the temperature control of the water bath was off while still stirred, so that the solution was slowly cooled down at room temperature. It took about 90 min for the temperature of the water bath to be cooled from 80°C to 35°C. The solution, which was in the white opaque state at 80°C, turned into a slightly pale transparent state around at 72°C, and the transparency persisted until the temperature fell to around 62°C. From around 62°C, the solution started to become white opaque again, turning into a completely white opaque state at 58°C. This change of state was reversible: the similar change of state was observed when the temperature was gradually raised from room temperature.

### 3.2: Examination on whether phospholipid alone or cholesterol alone can be dissolved

75. mg of DPPC and 40 mg of cholesterol were separately weighed into glass vials, to each of which a mixture of 4 mL of 10 wt/vol% sucrose solution and 0.85 mL of t-BuOH was added; and similar experiments as those in 3.1 were conducted. Where DPPC alone was used, the aqueous solution was in the transparent state between 80°C and 50°C, turning into a slightly pale transparent state at around 48°C. This state persisted until the temperature fell down to about 35°C, where the solution suddenly changed into a white opaque state. In contrast, where cholesterol alone was used, aggregates of cholesterol adhering to the vial wall were observed and the solution never became transparent at any temperature.

### 3.3 Examination of the t-BuOH concentration

32.7 mg of DPPC and 17.2 mg of cholesterol were put together into glass vials. t-BuOH at the volumes shown in Table 1 and 2 ml of 50% sucrose solution were mixed to the lipid mixture, and then pure water was added at a final volume of 10m L. Thus solutions containing t-BuOH at various concentrations were prepared.

**[Table 1]**

| t-BuOH final concentration (vol%) | t-BuOH volume (mL) | 50% sucrose volume (mL) | Volume after addition of pure water (mL) |
|---|---|---|---|
| 10 | 1 | 2 | 10 |
| 12 | 1.2 | 2 | 10 |
| 14 | 1.4 | 2 | 10 |
| 16 | 1.6 | 2 | 10 |
| 18 | 1.8 | 2 | 10 |
| 20 | 2.0 | 2 | 10 |
| 22 | 2.2 | 2 | 10 |
| 24 | 2.4 | 2 | 10 |
| 26 | 2.6 | 2 | 10 |
| 28 | 2.8 | 2 | 10 |

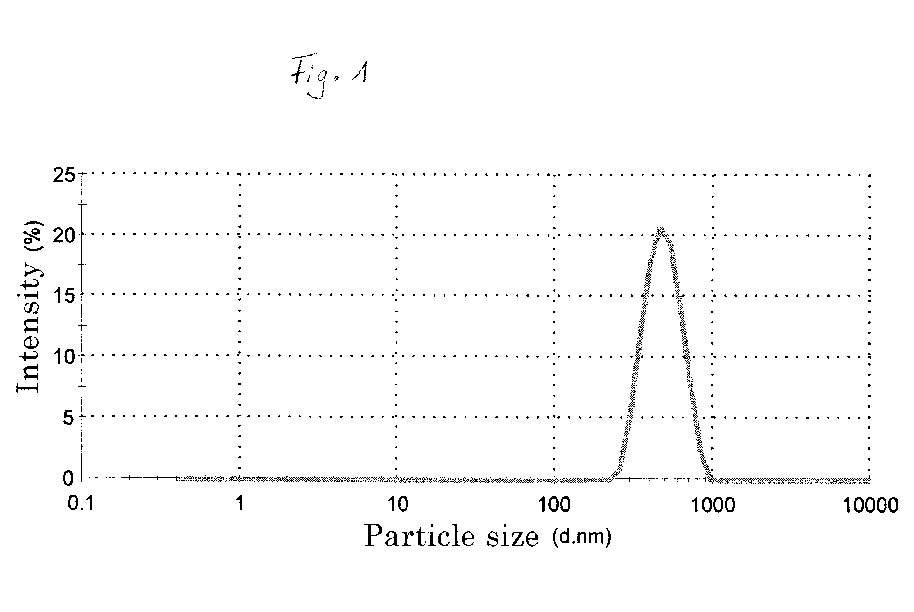

Each vial was stirred in a water bath at 90°C for 10 min, removed from the water bath, and then cooled with stirring at room temperature. After cooling, a part of the liposome suspensions was removed and diluted with PBS (or 10 wt/vol% sucrose solution). Particle size distributions were measured using the Zetasizer Nano ZS. The result is shown in Table 2. Additionally, as an example, the particle size distribution chart of the liposome suspension prepared under the conditions of the t-BuOH concentration of 16 vol% is shown in FIG. 1. In Table 2, the asterisk (*) indicates heterogeneous particles which do not meet the quality standard for particle diameter measurement established by Malvern.

**[Table 2]**

| t-BuOH concentration (vol%) | Z-Average (d.nm) | PDI | Result quality |
|---|---|---|---|
| 10 | 2338 | 1.000 | * |
| 12 | 442 | 0.196 | Good |
| 14 | 651 | 0.194 | Good |
| 16 | 498 | 0.195 | Good |
| 18 | 394 | 0.130 | Good |
| 20 | 1589 | 0.644 | * |
| 22 | 1324 | 0.529 | * |
| 24 | 1463 | 0.747 | * |
| 26 | 1160 | 0.708 | * |
| 28 | 1010 | 0.630 | * |

As shown in Table 2 and FIG. 1, liposomes extremely uniform in particle diameter could be successfully produced particularly in the t-BuOH concentration of 12 to 18 vol%.

### 3.4: Examination of the concentrations of 1-propanol, 2-propanol, 2-butoxyethanol, etc.

Preferred concentration ranges of the water-miscible solvents were examined by the same method as that in 3.3., except that t-BuOH was replaced with 1-propanol, 2-propanol, or 2-butoxyethanol. The result is shown in Tables 3 to 5.

**[Table 3]**

| 1-propanol concentration (vol%) | Z-Average(d.nm) | PDI | Result quality |
|---|---|---|---|
| 3 | 1107 | 0.759 | * |
| 4 | 2596 | 0.515 | * |
| 5 | 943.6 | 0.426 | Good |
| 7 | 349.1 | 0.222 | Good |
| 11 | 373.4 | 0.217 | Good |
| 15 | 949.1 | 0.302 | Good |
| 18 | 1130 | 0.206 | Good |
| 19 | 929.1 | 0.064 | Good |
| 20 | 1897 | 0.185 | * |

**[Table 4]**

| 2-propanol concentration (vol%) | Z-Average(d.nm) | PDI | Result quality |
|---|---|---|---|
| 12 | 10.49 | 0.608 | * |
| 13 | 355.7 | 0.2 | Good |
| 14 | 360.2 | 0.22 | Good |
| 18 | 551.3 | 0.29 | Good |
| 20 | 951.3 | 0.246 | Good |
| 24 | 1482 | 0.168 | Good |
| 26 | 1404 | 0.258 | Good |
| 27 | 1749 | 0.463 | * |
| 29 | 1303 | 0.527 | * |

**[Table 5]**

| 2-butoxyethanol concentration (vol%) | Z-Average(d.nm) | PDI | Result quality |
|---|---|---|---|
| 4 | 914.4 | 0.843 | * |
| 5 | 1287 | 0.440 | * |
| 6 | 1308 | 0.121 | Good |
| 7 | 930.7 | 0.187 | Good |
| 8 | 1382 | 0.103 | Good |
| 9 | 705.9 | 0.310 | Good |
| 10 | 1309 | 0.298 | * |
| 11 | 2898 | 1.000 | * |

As shown in Tables 3 to 5, liposomes extremely uniform in particle diameter could be successfully produced in the range of 5 to 19 vol% for 1-propanol, in the range of 13 to 26 vol% for 2-propanol, and in the range of 6 to 9 vol% for 2-butoxyethanol.

### 3.5: Examination in terms of changes in the lipid composition (1)

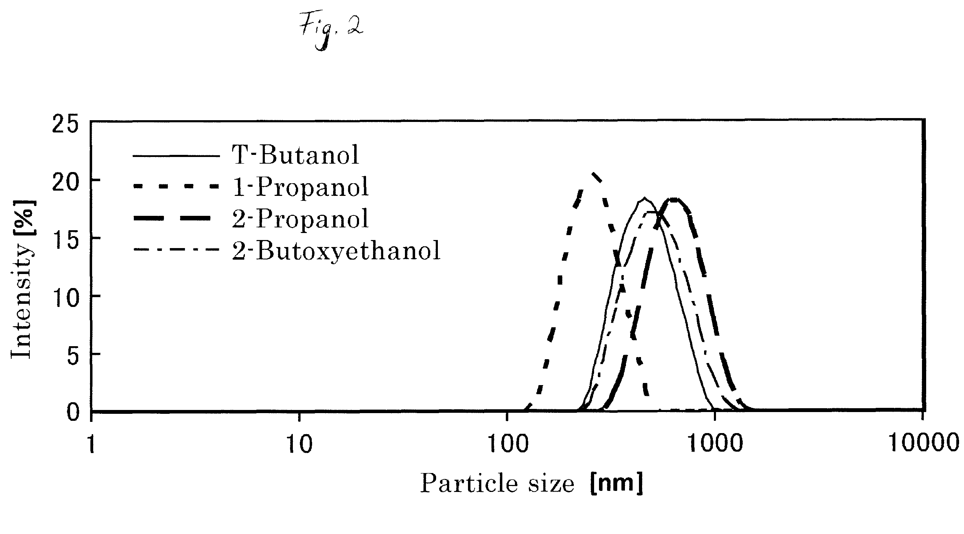

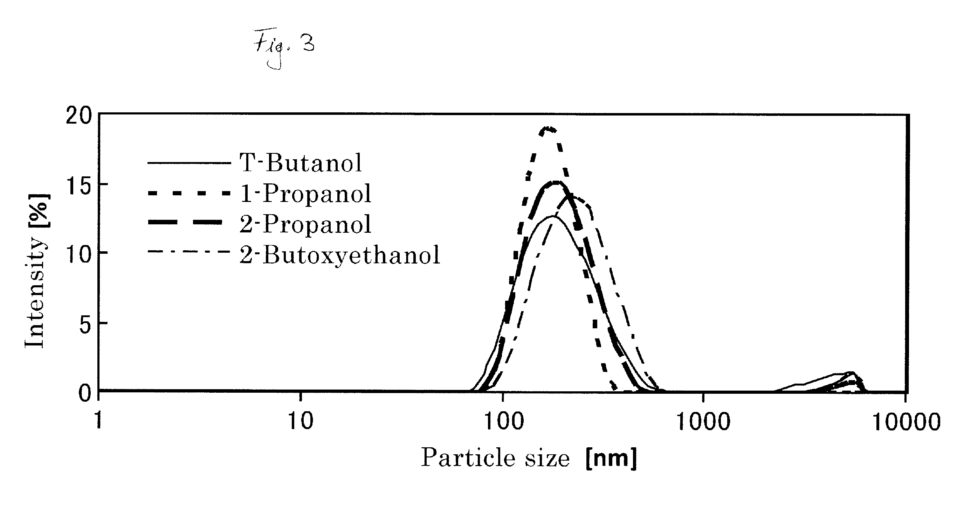

75.9 mg of DPPC, 40.0 mg of cholesterol, and 14.3 mg of stearylamine or 0.772 mg of DPPG were added to individual vials. Subsequently, 10 wt/vol% sucrose solution containing 17 vol% t-BuOH, 10 wt/vol% sucrose solution containing 17 vol% 1-propanol, 10 wt/vol% sucrose solution containing 25 vol% 2-propanol, or 10 wt/vol% sucrose solution containing 8 vol% 2-butoxyethanol was added to each vial at a volume of 4.85 mL each. Each vial was stirred in a water bath at 70°C for 30 min, and then cooled with stirring at room temperature. After cooling, a part of the liposome suspension was removed and diluted with PBS. Particle size distributions were measured using the Zetasizer Nano ZS. The results are shown in Tables 6 and 7 as well as FIGs. 2 and 3. Furthermore, the liposome suspensions were diluted with PBS, liposomes were precipitated by centrifugation, and the supernatant was replaced with PBS (i.e. washing by centrifugation). By repeating washing by centrifugation 3 times, the solutions dispersing liposomes were replaced with PBS, and the water-miscible organic solvent and the sucrose outside liposomes were removed. Then, after quantification of the cholesterol in the liposome suspensions, the content of the sugar (sucrose) which was used for the preparation of the liposomes and was encapsulated inside the liposomes was measured by the phenol-sulfuric acid method. The ratios of sugar encapsulation were obtained by the formula: (ratio of sugar concentration to cholesterol concentration after centrifugal washing)/ (ratio of the sugar concentration to cholesterol concentration before washing by centrifugation).

**[Table 6] Result with use of DPPC, cholesterol, and stearylamine**

| Solvent | Avera particle diameter [nm] | PDI | Sugar encapsulation ratio |
|---|---|---|---|
| 1-propanol | 248 | 0.059 | 0.188 |
| 2-propanol | 699 | 0.339 | 0.193 |
| 2-butoxyethanol | 493 | 0.100 | 0.264 |
| t-BuOH | 443 | 0.074 | 0.309 |

**[Table 7] Result with use of DPPC, cholesterol, and DPPG**

| Solvent | Average particle diameter [nm] | PDI | Sugar encapsulation ratio |
|---|---|---|---|
| 1-propanol | 165 | 0.066 | 0.212 |
| 2-propanol | 185 | 0.171 | 0.313 |
| 2-butoxyethanol | 228 | 0.228 | 0.396 |
| t-BuOH | 191 | 0.235 | 0.435 |

As shown in Tables 6 and 7 as well as FIGs. 2 and 3, where t-BuOH or 2-butoxyethanol is used as the water-miscible organic solvent, almost the same particle diameter and sugar encapsulation ratio were obtained. In particular, where t-BuOH is used, sucrose (sugar) used for the liposome preparation was encapsulated into liposomes at an extremely high ratio of 40% or more. This strongly suggests that, by dissolving a substance to be encapsulated into the solution for liposome preparation, the substance is encapsulated in the liposome at an extremely high ratio, as with sucrose.

### 3.6: Examination in terms of changes in the lipid composition (2)

Mixture (1) was prepared by putting 76 mg of DPPC and 40 mg of cholesterol into a vial and then adding 4 mL of 10 wt/vol% sucrose solution and 0.85 mL of t-BuOH. Mixtures (2) and (3) were prepared by putting 76 mg of DPPC, 40 mg and 30 mg of cholesterol, respectively, and 0.77 mg of DPPG into vials and then adding 4 mL of 10 wt/vol % sucrose solution and 0.85 mL of t-BuOH. Mixture (4) was prepared by putting 67.7 mg of DPPC, 40.6 mg of cholesterol, 9.1 mg of DPPE, and 7.3 mg of DCP into a vial and then adding 4 mL of 10 wt/vol% sucrose solution and 0.85 mL of t-BuOH. Mixture (5) was prepared by putting 76 mg of DPPC, 40 mg of cholesterol, and 14.3 mg of SA into a vial and then adding 4 mL of 10 wt/vol% sucrose solution and 0.85 mL of t-BuOH. Mixture (6) was prepared by putting 76 mg of HSPC and 40 mg of cholesterol into a vial and then adding 4 mL of 10 wt/vol% sucrose solution and 0.85 mL of t-BuOH. Each vial was stirred in a water bath at 70°C for 30 min, and then cooled with stirring at room temperature. After cooling, an aliquot of the liposome suspension was removed and diluted with PBS. Particle size distributions were measured to obtain average liposome particle size using the Zetasizer Nano ZS. The result is shown in Table 8.

**[Table 8]**

| Mixture | Lipid composition (molar ratio) | Average particle size of liposomes (Z-Average, nm) |
|---|---|---|
| (1) | DPPC:Chol.=1:1 | 490 |
| (2) | DPPC:Chol.:DPPG=1:1:0.01 | 260 |
| (3) | DPPC:Chol.:DPPG=1:0.75:0.01 | 420 |
| (4) | DPPC:Chol.:DPPE:DCP=41:47:6:6 | 180 |
| (5) | DPPC:Chol.:SA=1:1:0.51 | 364 |
| (6) | HSPC:Chol. =76:40 (weight ratio) | 279 |

The result revealed that the average particle size varied depending on the lipid composition.

### 3.7: Examination of cooling rate

A solution containing 17.5 vol% t-BuOH was prepared by adding 1.75 mL of t-BuOH and 2 mL of 50% sucrose aqueous solution, and then adding pure water to make its final volume 10 mL. Next, 32.7 mg of DPPC and 17.2 mg of cholesterol were put into glass vials. 2 mL of the solution containing 17.5 vol% t-BuOH was added to the lipid mixture. The vials were stirred for 10 min in a water bath maintained at 70°C. Subsequently, the vials were cooled at a cooling of 1°C/min by transferring them to water baths with the temperature at 50°C, 40°C, and 30°C,. When the temperatures of the vials became 50°C, 40°C, and 30°C, aliquots of the liposome suspension were removed, diluted with PBS, and subjected to particle size distribution measurement using the Zetasizer Nano ZS. Further, for comparison, a vial containing the same lipid mixture as the above, to which 2 mL of the solution containing 17.5 vol% t-BuOH was added, was stirred for 10 min in a water bath maintained at 80°C. With still stirring, temparature control of the water bath temperature was turned off and the vial was slowly cooled down at room temperature. Cooling from 80°C to 35°C required 90 min (cooling rate: 0.5°C/min). When the temperature of the vial became 35°C, an aliquot of the liposome suspension was removed, diluted with PBS, and subjected to particle size distribution measurement using The Zetasizer Nano ZS. The result is shown in Table 9.

**[Table 9]**

| Temperature (°C) | Z-Average (d.nm) | PDI | Result quality |
|---|---|---|---|
| 50 (Rapid cooling) | 527.9 | 0.240 | Good |
| 40 (Rapid cooling) | 516.8 | 0.217 | Good |
| 30 (Rapid cooling) | 517.1 | 0.217 | Good |
| 35 (Slow cooling) | 1014 | 0.577 | * |

As shown in Table 9, the liposomes in the liposome suspension rapidly cooled down to 50°C, 40°C, and 30°C were highly uniform in particle diameter with an average particle diameter of about 500 nm. The uniformity of the liposomes in the liposome suspension treated by slow cooling were low, compared with the aforementioned three groups of liposomes.

### INDUSTRIAL APPLICABILITY

The liposomes produced by the present invention can potentially be applied to, for example, DDS, microcapsules for cosmetics, and the like.

## Claims

1. A method for producing a liposome comprising the steps of:
heating a mixture containing one or more lipids, water, and an organic solvent; and
cooling the mixture after the heating step.

2. The method for producing a liposome of claim 1, wherein the mixture is a mixture of the lipids and an aqueous solution comprising a water-miscible organic solvent.

3. The method for producing a liposome of claim 1 or 2, wherein the aqueous solution comprises 5 to 30% by volume of the water-miscible organic solvent, relative to the total volume of the solution.

4. The method for producing a liposome of any one of claims 1 to 3, wherein the water-miscible organic solvent comprises one or more organic solvents selected from t-butanol, 1-propanol, 2-propanol, and 2-butoxyethanol.

5. A method for dissolving cholesterol, comprising the steps of:
heating a mixture obtained by mixing cholesterol with a phospholipid and an aqueous solution comprising a water-miscible organic solvent.

6. The method for dissolving cholesterol of claim 5, wherein the aqueous solution comprises 5 to 30% by volume of the water-miscible organic solvent, relative to the total volume of the solution.

7. The method for dissolving cholesterol of claim 5 or 6, wherein the water-miscible organic solvent comprises one or more organic solvents selected from t-butanol, 1-propanol, 2-propanol, and 2-butoxyethanol.

8. A method for selecting an organic solvent which can be used for producing a liposome, comprising the steps of:
producing a liposome by using the method of any one of claims 1 to 3 with a candidate organic solvent; and
examining whether or not the liposome is uniform in size.
